# EUROPEAN PATENT SPECIFICATION

(11) **EP 0 642 504 B1**
(45) Date of publication and mention of the grant of the patent: **15.12.1999**
(21) Application number: 92920778.5
(22) Date of filing: 11.09.1992
(51) Int. Cl.: C07D 311/84, A61K 31/35, C07D 311/80

(54) **(3S,4S)-DELTA-6-TETRAHYDROCANNABINOL-7-OIC ACIDS AND DERIVATIVES THEREOF, PROCESSES FOR THEIR PREPARATION AND PHARMACEUTICAL COMPOSITIONS CONTAINING THEM**
(3S, 4S)-DELTA-6-TETRAHYDROCANNABINOL-7-SÄUREN UND IHRE DERIVATE, VERFAHREN ZU IHRER HERSTELLUNG UND SIE ENTHALTENDE PHARMAZEUTISCHE ZUSAMMENSETZUNGEN
ACIDES (3S,4S)-DELTA-6-TETRAHYDROCANNABINOL-7-OIQUES ET DERIVES DE CES ACIDES, PROCEDES POUR LEUR PREPARATION ET COMPOSITIONS PHARMACEUTIQUES CONTENANT CES ACIDES

(30) Priority: 12.09.1991 IL 99468
(43) Date of publication of application: 15.03.1995
(73) Proprietor: YISSUM RESEARCH AND DEVELOPMENT COMPANY OF THE HEBREW UNIVERSITY OF JERUSALEM, 91 999 Jerusalem (IL)
(72) Inventor: MECHOULAM, Raphael, Jerusalem (IL); BREUER, Aviva, Jerusalem (IL); DEVANE, William, Jerusalem (IL); BURSTEIN, Summer, H., Framingham, MA 01701 (US)
(74) Representative: Gardner, Rebecca
(86) International application number: US9207718
(87) International publication number: WO9305031

(56) References cited:
- EP-A- 0 276 732
- WO-A-89/12446
- US-A- 5 036 014
- US-A- 5 144 030
- JOURNAL OF MEDICINAL CHEMISTRY vol. 35, no. 17 , 21 August 1992 , WASHINGTON US pages 3135 - 3141 S. H. BURSTEIN ET AL. 'SYNTHETIC NONPSYCHOTROPIC CANNABINOIDS WITH POTENT ANTINFLAMMATORY,ANALGESIC,AND LEUKOCYTE ANTIADHESION ACTIVITIES.'
- J.O.C., 1986, SCHWARTZ et al., 51 (26), 5463-5; see compounds 3a and 3c.

## Description

### FIELD OF THE INVENTION

The present invention relates to (3S,4S)-delta-6-tetrahydrocannabinol-7-oic acids and homologs and derivatives thereof, essentially free of the (3R,4R) form, and to processes for their preparation. The invention also relates to pharmaceutical compositions containing said compounds as active ingredients, having anti-inflammatory, analgetic, leucocyte anti-adhesion, antiplatelet activating factor (PAF), and anti-glaucoma activities, as well as properties effective in alleviating certain symptoms due to neuronal injury or loss.

### BACKGROUND OF THE INVENTION

The (3S,4S) enantiomers of cannabimimetically active cannabinoids, such as the natural (3R,4R)-delta-1-tetrahydrocannabinol (THC) are generally not cannabimimetic. This lack of undesirable CNS side effects makes them suitable candidates as therapeutic agents. It has been previously shown that the (3S,4S)-7-hydroxy-delta-6-tetrahydrocannabinol 1,1-di-methylheptyl homolog (compound la in the appended Figures) is an analgetic, entiemetic and anti-NMDA drug [U.S. Patent 4,876,276 and co-pending Israel Patent Application No. 92238; Mechoulam, R., et al., Tetrahedron:Asymmetry, 1, 315(1990); Feigenbaum, J.J., et al., Proc. Natl. Acad. Sci., 86, 9584 (1989)]. It has also been shown that (3R,4R)-delta-l-tetrahydrocannabinol-7-oic acid (compound 2), which also shows no psychotropic effects, is an anti-inflammatory and analgetic compound [U.S. Patent 4,847,290]. However, the (3S,4S)-tetrahydrocannabinol-7-oic acids have not yet been prepared and their therapeutic activity has been unknown so far. These compounds have now been synthesized and were found to possess unexpected therapeutically important properties.

### SUMMARY OF THE INVENTION

The present invention relates to (3S,4S)-delta-6-tetrahydrocannabinol-7-oic acids, homologs and derivatives thereof having the general formula: 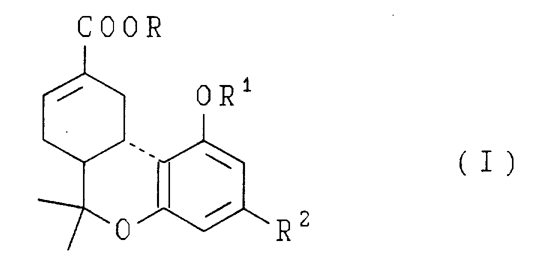 wherein R is a hydrogen atom or a C₁-C₅ alkyl group, R¹ is a hydrogen atom or a C₁-C₅ acyl group and R² is selected from the group consisting of: (a) a straight-chained or branched C₅₋ C-₁₂ alkyl; (b) a group -O-R₄, wherein R₄ is a straight-chained or branched C₂-C₉ alkyl which may be substituted at the terminal carbon atom by a phenyl group; and (c) a group -(CH₂)ₙ-O-alkyl, where n is an integer of from 1 to 7 and the alkyl group contains from 1 to 5 carbon atoms, with the exception of 6a,7,10,10a tetrahydro-6,6-dimethyl-9-carboxyl-3-pentyl-6H-dibenzo[b,d]pyran-1-ol

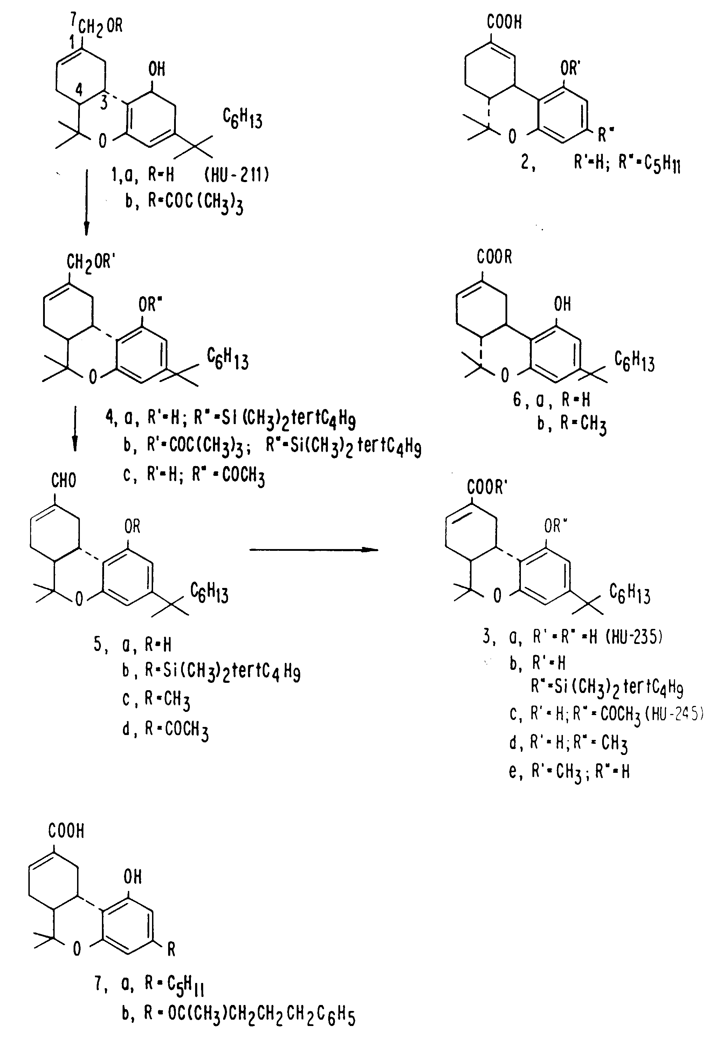

The most preferred compounds are the 1,1-dimethylheptyl (DMH) homologs of (3S,4S)-(+)-delta-6-tetrahydrocannabinol-7-oic acid (hereafter designated HU-235, compound 3a in the appended Fig. 1) and its acetate (hereafter designated HU-245, compound 3c in Fig. 1).

It is stressed that all the compounds are of the (+)-(3S,4S) configuration, essentially free of the (-)-(3R,4R) enantiomer. The compounds of the type defined by general formula (I) are substantially devoid of "cannabis-type" CNS activity.

The invention also relates to processes for the preparation of compounds of general formula (I).

In a first embodiment the process for the preparation of a compound of formula (I) wherein R is a hydrogen or a C₁-C₅ alkyl group and R¹ and R² are as defined in claim 1, comprises:
(a) reacting a compound of the formula: 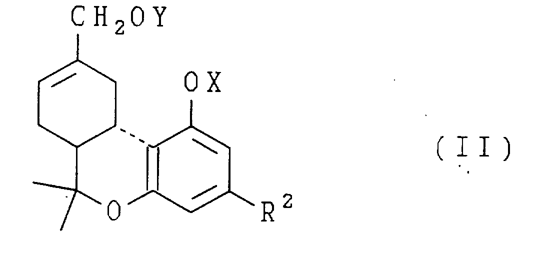 wherein Y is a straight-chained or branched C₁-C₅ alkyl, X is a suitable protective group and R² is as defined in formula I with a suitable reducing agent to give the corresponding allylic alcohol of formula III: 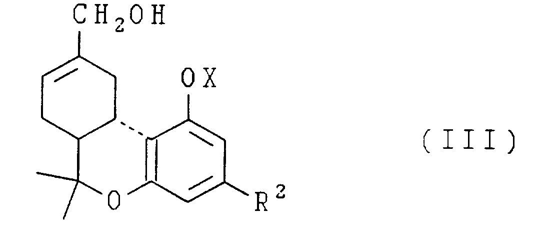
(b) oxidizing the alcohol of formula III with a suitable oxidizing agent to give the corresponding aldehyde of formula IV: 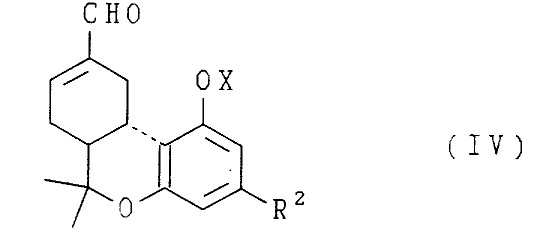
(c) oxidizing the aldehyde of formula IV with a suitable oxisizing agent to give the corresponding allylic acid of formula V: 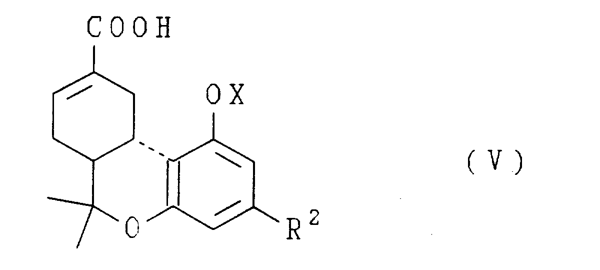 and
(d) removing the protective group X to give the acid according to formula (I).

Y can be, for example, (trimethyl)methyl.

In stage (a) the protective group OY may optionally be replaced by the group -NR⁴R⁵, wherein R⁴ and R⁵ are C₁-C₅ alkyl groups.

The phenolic protective groups X can be, for example, dimethyl-tert.-butylsilyl, or other silyl derivatives such as phenylsilyl, a C₁-C₅ alkyl group or benzyl. These alkyl or benzyl ethers, which serve as protective groups to the phenol ring, can later be removed by standrd procedures.

The reducing agent can be, for example, lithium aluminum hydride.

In a second embodiment compounds of general formula (I) wherein the substituents are as defined above may be prepared as follows:
(a) subjecting a compound of the formula: 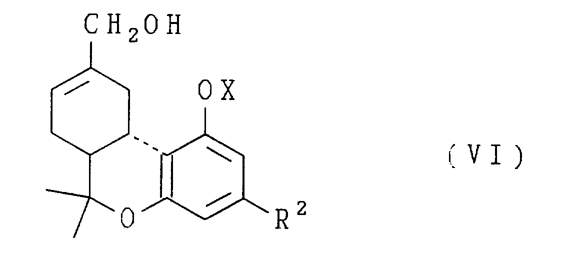 wherein R² is as defined above to selective esterification, to give the corresponding phenolic ester of formula VII: 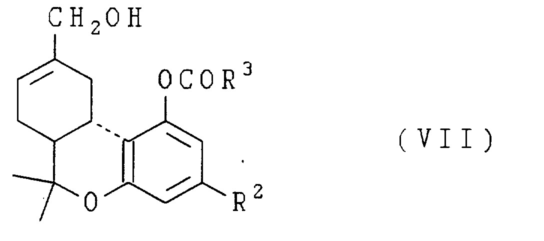 wherein R³ is a C₁-C₅ alkyl, or benzyl which may be substituted at the para position by chlorine or bromine;
(b) oxidizing the ester of formula VII with a suitable oxidizing agent to give the corresponding aldehyde of formula VIII: 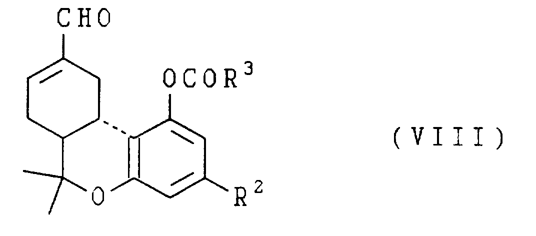
(c) oxidizing the aldehyde of formula VIII with a suitable oxidizing agent to give the corresponding acid of formula I: 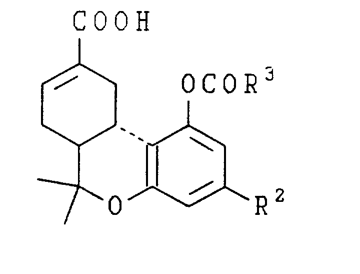 and
   optionally removing the ester group COR³ to give the corresponding compound:

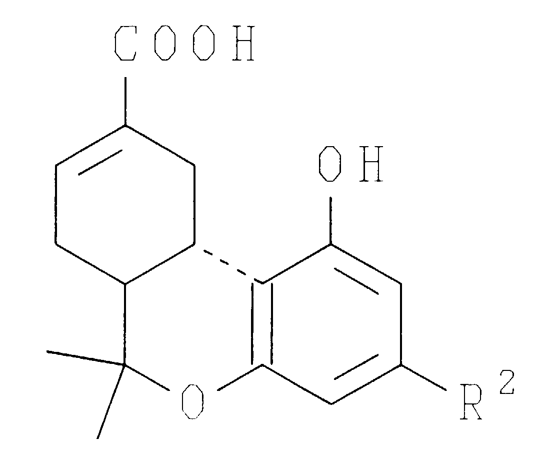

According to a first embodiment, the pathway of which is illustrated in Fig. 1, the starting material is 7-hydroxy-delta-6-tetrahydrocannabinol-DMH(1a)(HU-211) (which may be prepared according to the procedure reported by Mechoulam, R., et al., Tetrahdron: Asymmetry, 1, 315 (1990)), protected at the 7-position by conventional protective groups, for example pivalate (compound 1b), the phenolic group also being protected by a conventional protective group, for example dimethyltert.butylsilyl (compound 4b). This protected starting material is reduced, for example, by lithium aluminum hydride, to give the corresponding allylic alcohol (compound 4a). The alcohol is then oxidized, for example with chromic oxide in pyridine, to give the corresponding aldehyde (compound 5b). Other oxidizing agents, for example, tert.-butyl chromate, chromic acid/pyridine complex or related chromate derivatives or manganese dioxide, particularly in the presence of cyanide, or related manganese derivatives may be used. The aldehyde is further oxidized, for example with sodium chlorite, to give the corresponding allylic acid (compound 3b). Other oxidizing agents such as manganese dioxide in the presence of sodium cyanide and acetic acid and related manganese derivatives may be used. The phenolic protective group is then removed, to give the desired acid (compound 3a, HU-235). This synthetic route may yield a series of related compounds which were prepared.

The second embodiment also uses 7-hydroxydelta-6-tetra-hydrocannabinol-DMH (Compound la) as the starting material. This starting material is subjected to selective esterifacation to give the monoacetate (compound 4c), which on oxidation with, for example, chromic acid in pyridine gives the aldehyde (compound 5d), which is further oxidized to the acid (compound 3c, HU-245). Other oxidizing agents, as above, may be used. Removal of the phenolic protective group leads to the desired acid (compound 3a, HU-235).

The invention also relates to pharmaceutical compositions which possess potent analgetic, anti-inflammatory, anti-emetic, anti-galucoma, anti-platelet activating factor and leukocyte anti-adhesion activities, containing compounds of general formula (I) as active ingredient. These compositions also reduce and may even prevent excitatory amino acid neurotoxicity due to acute injury to the central nervous system, such as injuries due to prolonged epileptic seizures, compromised or reduced blood supply, deprivation of glucose supply and mechanical trauma. The compositions are of special value in grand mal seizure, global hypoxic ischemic insults, in hypoxia, alonr or in combination with blood flow reduction (ischemia), as well as in cases of abrupt occlusion of cerebral arteries (stroke). The compositions of the present invention may also be effective in alleviating certain chronic degenerative diseases associated with gradual selective neuronal loss, mainly Huntington's chorea, Parkinsonism and Alzheimer's disease. The compositions of the present invention have also been discovered to be effective in the treatment of multiple sclerosis. The compositions of the present invention are also useful in the treatment of poisoning affecting the central nervous system, for example strychnine, picrotoxin or organophosphorous poisoning.

The novel compositions contain in additin to the active ingredient conventional pharmaceutically acceptable carriers, diluents and the like. Solid compositions for oral administration such as tablets, pills, capsules or the like may be prepared by mixing the active ingredient with conventional, pharmaceutically acceptable ingredients such as corn starch, lactose, sucrose, sorbitol, talc, stearic acid, magnesium stearate, dicalcium phosphate and gums with pharmaceutically acceptable diluents. The tablets or pills can be coated or otherwise compounded with pharmaceutically acceptable materials known to provide a dosage form affording prolonged action or sustained release. Other solid compositions can be prepared as suppositories for rectal administration. Liquid forms may be prepared for administration or for injection, the term including sub-cutaneous, transdermal, intravenous, intratechal, etc. administration. The liquid compositions include aqueous solutions, flavored syrups, aqueous or oil suspensions, flavored emulsions with edible oils, as well as elixirs and similar pharmaceutical vehicles. In addition, the compositions of the present invention may be formed as aerosol, for intra-nasal and like administration.

The active dose for humans is generally in the range of from 0.005 mg to about 50 mg per kg body weight, in regimen of 1-4 times a day. However, administration every two days may also be possible, as the drug has a rather prolonged action. The preferred range of dosage is from 1.0 mg to about 20 mg per kg body weight. However, it is evident to the man skilled in the art that dosages would be determined by the attending physician, according to the disease to be treated, method of administration, patient's age, weight, counterindications and the like.

All the compounds defined above are effective in treating the above conditions and can be used as active ingredients of pharmaceutical compositions for treatment of one, or simultaneously several, of the symptoms or disorders defined above. The effective dosages are essentially similar.

The invention also relates to use of the compositions of the present invention for the treatment of the various pathological conditions described above. Administration of therapeutically effective amounts of the compositions of the present invention as used herein encompasses oral, parenteral, intravenous, intramuscular, sub-cutaneous, transdermal, intratechal, rectal and intra-nasal administration.

### PREPARATORY EXAMPLES

### 1. Synthesis of the dimethyl-tert-butylsilyl ether (4a).

The ester (1b) (2.9g, 6.17 mmoles), [a]D +152.6° (c, 17.2 mg/ml, CHCl₃), was dissolved in dry dimethylformamide (DMF) (6 ml.). Dimethyl-tert-butylsilyl chloride (1.85 g, 12.27 mmoles) and imidazole (1.67 g, 24.6 mmoles) were added and the resulting mixture was stirred for 48 hours at 38°C. Water (30 ml) was added and the mixture was extracted with ether. After evaporation of the dried ether layer an oil (4b) (3.6 g) was obtained; [α]D +153° (c, 24.45 mg/ml, CHCl₃); I.R. max (neat) 1725 cm-¹. No free hydroxyl groups were observed; ¹H NMR δ (CDCl₃) 3.28 (1 H, br d, J=16 Hz, C-2 equat. H), 4.46 (2 H, s, C-7H), 5.70 (1 H, m, C-6 H), 6.38 (1 H, d, J-1.5 Hz, arom.), 6.42 (1 H, d, J=1.5 Hz, arom). This oil (compound 4b) was used in the next step with no further purification.

A solution of compound (4b) (3.2 g, 5.5 mmoles) in dry ether (50 ml) was added under a nitrogen atmosphere to lithium aluminum hydride (870 mg) in dry ether (60 ml). The resulting mixture was boiled under reflux for 1.5 hours. After the usual work up (ethyl acetate followed by slow addition of a saturated solution of magnesium sulphate until a clear ether supernatant is formed) the ether layer was dried and evaporated to give an oil (3.2 g). The oil was chromatographed on a silica gel column (100 g), using ether:petroleum ether (6:4) as eluent, to give the alcohol (4a) (8 g, 67%); [α]D +175°; (7.6 mg/ml, CHCl₃) I.R. max (neat) 3320 cm-¹ (OH band); no carbonyl bands; ¹H NMR δ (CDCl₃) δ 3.38 (1 H, br d, J=16 Hz, C-2 equat. H) 4.02 (2 H, s, C-7 H), 5.72 (1 H, br d, C-6 H), 6.36, 6.42 (2 H, s, aromatic).

### 2. Synthesis of aldehyde (5b).

Dry pyridine (2.3 ml), followed by chromic oxide (1.44 g, 14.4 mmoles) were added to a solution of methylenechloride: DMF (4:1) (36 ml). The mixture was stirred for 15 min. The primary allylic hydroxy compound (4a) (1.8 g, 3.6 mmoles) in methylchloride: DMF (4:1) (7.2 ml) was added and the reaction mixture was stirred at room temp. for 1 hr. Ethanol (1.8 ml) was added, the mixture was stired for additional 10 min., and was then diluted with ethylacetate (180 ml). The resulitng mixture was filtered through a sintered glass funnel, packed with silica (3 cm), with a layer of anhydrous sodium sulfate on top, and eluted with ethyl acetate (ca 600 ml). The ethyl acetate filtrate was washed with dilute hydrochloric acid (1N), then with sodium bicarbonate solution and water. After evaporation of the dried organic solvent a semi-solid compound (5b) (1.7 g, 95%) was obtained. Crystallization from pentane gave the aldehyde (5b); m.p. 80-81°C; [α] -268° (C, 6.82 mg/ml, CHCl₃); I.R. max 1690cm-¹ (neat); ¹H NMR δ (CDCl₃) 3.82 (1 H, br d, J=15 Hz, C-2 equat. H), 6.38 and 6.42 (2H, s, aromatic), 6.80 (1 H, m, C-6 H), 9.50 (1 H, s, c-7 H). Anal. (C₃₁H₅₀O₃Si) C H.

### 3. Synthesis of (3R,4R)-delta-6-THC-DMH-7-oic acid (3a).

Sodium chlorite (488 mg) was added portionwise with vigorous stirring to a mixture of the aldehyde (5b) (498 mg, 1 mmole), 2-methyl-2-butene (2.24 ml), saturated aqueous potassium dihydrogenphosphate (1.34 ml) and t-butanol (22 ml). The reaction mixture was stirred at room temp. for 5 hrs. Water (20 ml) was added and the mixture was extracted several times with enthyl acetate, dried and evaporated to give the crude acid which was purified on a silica gel column (10 g, elution with 10% ether:pet.ether) to give the acid (3b) (460 mg, 89%) as an oil; [α]^{D} +218° (c, 13.7 mg/ml, CHCl₃) ; I.R. max 1680, cm-¹; and a broad band in the 2800-3600 region; ¹H NMR δ 3.75 (1 H, br d, J=18 Hz, C-2 equat. H), 6.23 (1 H, d, J=1.5, arom.), 6.27 (1 H, d, J=1.5, arom.), 7.00 (1 H, m, C-6 H).

Tetrabutylammonium fluoride (0.6 mmoles solution in THF) was added by injection under a nitrogen atmosphere to a cold solution (ice bath) of the acid (3b) (280 mg, 0.54 mmoles) in tetrahydrofuran (THF) (3 ml). The resulting solution was stirred at 0° for 15 minutes. Water was added and the mixture extracted several times with ether. The ether layer was dried and evaporated to give the crude product. The product was further purified on a silica gel column with ether:petroleum ether (1:1) as eluent. The solid thus obtained (140 mg, 56%) was crystallized from acetronitrile to give the acid (3a) m.p. 112-114 (sintering); [α]^{D} +275° (c, 3.8 mg/ml, CHCl₃), I.R. max (Nujol) 1680 cm-¹ and a broad band in the 3100-3600 region; ¹H NMR δ 3.82 (1 H, br d, J=18 Hz, C-2 equat.H), 6.22 (1 H, d, J=1.5 Hz, arom.) 6.38 (1 H, d, J=1.5 arom). 7.16 (1 H, m, C-6 H); m/z 400 (M). The acetate (3c), (HU-245), melts at 120-122°; [α]^{D} +265° (c, 9.0 mg/ml, CHCl₃) I.R. max (Nujol) 1760 cm-¹ and a broad band in the 3100-3600 cm-¹ region; ¹H NMR (CDCl₃) δ 2.30 (3 H, s, OCOCH₃), 3.38 (1 H, br d, J=19 Hz, C-2 equat. H), 6.56, (1 H, d, J=1.5 Hz arom.) 6.68 (1 H, d, j=1.5 arom.), 7.18 (1 H, m, C-6 H). Anal. (C₂₇H₃₈O₅) C, H. The methyl ether (3d) melts at 172-174°; [α]^{D} +270° (7.5 mg/ml, CHCl₃) ; I.R. max (KBr) 1660, 1680 cm-¹ and a broad band in the 3100-3600 region; (CHCl₃) 1696 cm-¹; ¹H NMR (CDCl₃) 3.76 (1 H, br d, J=18 Hz, C-2 equat. H), 3.80 (3 H, s, OCH₃), 6.39, 6.42, (2 H, s, aromatic), 7.16 (1 H, m, C-6H); MS m/z 414 (M⁺). Anal. (C₂₆H₃₈O₄) C, H.

### 4. Synthesis of aldehyde (5a).

Tetrabutylammonium fluoride (0.4 mmoles, soln. in THF) was added, by injecton to a cold solution (ice bath) of the aldehyde (5b) (200 mg, 0.4 mmoles-) in THF (4 ml), under a nitrogen atmosphere. The solution was stitred at 0°C for 5 minutes and then at room temperature for 15 minutes. The solvent was evaporated and the residue was separated on a silica gel column (10 g). The product was eluted with ether:petroleum ether (15:85). The solid obtained (120 mg, 78%) was crystallized from pentane to give the required compound (5a) m.p. 174-175°; I.R. max. (KBr) 1690 cm-¹; ¹H NMR (CDCl₃) δ 3.84 (1 H, d, J=17 Hz, C-2/equat. H), 6.24, 6.36 (2 H, s, aromatic), 6.86 (1 H, m, C-6 H), 9.48 (1 H, s, C-7 H). Anal. (C₂₅H₃₆O₃) C, H. The methyl ether (5c) melts at 109-110°; [α]^{D} +302 (c, 8.2 mg/ml, CHCl₃); I.R. max (neat) 1680 cm-¹; ¹H NMR (CDCl₃) δ 3.76 (1 H, d, J=18 Hz, C-2 equat. H), 3.80 (3 H, s, OCH3), 6.38, (1 H, d, J=1.5, arom.), 6.42 (1 H, d, J=1.5, arom.), 6.82 (1 H, m, C-6 H), 9.50 (1 H, s, C-7 H); MS m/z 398 (M⁺). Anal. (C₂₆H₃₈O₃) C, H.

### 5. Synthesis of HU-211, monoacetate (4c).

Potassium (1 g) was added to 2-methyl-2-butanol (16 ml) under a nitrogen atmosphere. The mixture was warmed, at 80°, until all the metal had reacted. The excess alcohol was removed by vacuum distillation and the dry residue was dissolved in dry benzene (45 ml). Two ml of this solution were added to HU-211 (300 mg, 0.78 mmoles) dissolved in dry benzene (30 ml). The solution was stirred, under a nitrogen atmosphere, for 2 hrs., then it was washed with a dilute HCl solution (1N), followed by a sodium bicarbonate solution (1N) and then with water. The organic layer was dried over MgSO₄, and evaporated. The oil obtained was chromatographed on silica gel (15 gr.) Elution with ether:petroleum ether (1:10) gave the diacetate of HU-211 (99 mg) followed by traces of the monoacetate (on the allylic alcohol moiety), eluted with ether:petroleum ether (2:10), followed by the monoacetate (4c), 150 mg, identified by the I.R. peak at 1760 cm-¹; ¹H NMR δ 2.28 (3H, s, OCOCH₃), 2.98 (1H, dd, C-2 equat. H), 4.02 (2H, q, C-7 H), 5.72 (1H, d, C-6 H), 6.52, 6.66 (arom. H).

### 6. Synthesis of aldehyde (5d).

Dry pyridine (0.645 ml, 8 mmol) was added to methylene chloride:DMF (4:1), followed by chromic oxide (400 mg, 4 mmol). The mixture was stirred at toom temp. for 15 min. The ester (4c) (430 mg, 1 mmol) in the above solvent mixture (2 ml) was added and the reaction mixture was stirrred at room temperatue for 16 hrs., then diluted with ethyl acetate (50 ml) and filtered through a sintered glass funnel covered with a 3 cm layer of silica, with a layer (1 cm) of anhydrous magnesium sulphate over it. Ethyl acetate (200 ml) was passed through this double layer; the solvents were evaporated and the residue was chromatographed on silica, using ether:petroleum ether (1:5), yielding the aldehyde (5d) 280 mg (66%) as an oil, identified by the I.R. peaks at 1760 cm-¹ (phenolic acetate) and 1680 cm-¹ (unsaturated aldehyde); ¹H NMR δ 2.20 (3H, s, OCOCH₃), 2.82 (1H, dd, C-2 equat. H), 6.42, 6.58 (arom. H), 6.70 (C-6 H), 9.40 (aldehyde H). This aldehyde was used without further purification.

### 7. Synthesis of (3S,4S)-THC-DMH-7-oic acid, acetate (3c) (HU-145).

Sodium chlorite (488 mg, 4.3 mmol) was added portionwise with vigorous stirring to a mixture of the aldehyde (5d) (4.26 mg, 1 mmole), 2-methyl-2-butene (2.24 ml, 21 mmole), saturated sodium hydrogen phosphate (1.34 ml) and t-butanol (22 ml). The reaction mixture was stirred for 5h. at room temp., extracted with ethyl acetate, dried over magnesium sulphate and the solvent was removed. The acid obtained (390 mg, 88%), m.p. 120-122° (from pentane) was identical to the material described above.

### PHARMACOLOGICAL EXAMPLES

### 1. Relief of Edema Induced by Arachidonic Acid and PAF.

The induction of paw edema in rodents by injection of arachidonic acid has been used as an experimental model for inflammation [Calhoun, W., et al., Agents and Actions, 21, 306 (1987)]. Prior administration of non-steroidal anti-inflammatory drugs (NSAIDs) in many cases leads to a dose related inhibition of the edematous response which may be considered a predictor of clinical efficacy. The cannabinoid acid HU-235, and related compounds were effective in reducing paw edema in this model as seen in Table 1.

A second model, in which edema is induced by platelet activating factor (PAF), was used to evaluate activity. As may be seen from the results summarized in Table II, the acid HU-235 and related compounds were found to be active.

The conditions were based on those reported previously [Calhoun et al., ibid.]. Water was substituted for mercury as the displacement medium and was found to give satisfactory results. PAF (1.0 µg) or arachidonic acid (1.0 mg) dissolved in 50 µl of 5% ethanol in saline, were injected s.c. into the plantar surface of the right hind paw of CD-1 female mice (20-25 g) obtained from Charles River Labs. The mice were under ether anesthesia during this procedure. The volume of the right foot was measured to the level of the lateral malleous by water displacement before treatment and 15 min after PAF injection or 30 min after archidonate injection. The change in paw volume was calculated for each mouse and the significance for each group was determined by a paired t test analysis.

**Table I**

| Inhibition of Arachidonic Acid-Induced Paw Edema¹ | | | | |
|---|---|---|---|---|
| Dose (mg.kg)² | HU-245 (3c) | 7a | 7b | HU-235(3a) |
| 0.050 | 35.7 (N.S.) | 5.5 (N.S.) | 48.5* | 42.1 (N.S.) |
| 0.100 | 50.1* | 10.2 (N.S.) | 66.2* | 65.8* |
| 0.250 | 48.2* | 40.1 (N.S.) | 51.4* | 52.6* |
| 0.55 | 42.2 (N.S.) | 47.2* | 48.2* | 47.4 |

| | | | | |
|---|---|---|---|---|
| ¹Values shown are percent inhibition of paw edema when compared to vehicle treated controls. | | | | |
| *95% significance by ANOVA. N.S. - nonsignificant. | | | | |
| ²Control mice were given peanut oil (50 µl) orally. | | | | |

**Table II**

| Inhibition of PAF-Induced Paw Edema³ | | | | | | |
|---|---|---|---|---|---|---|
| Treatment | Dose mg/kg⁴ | | | | | |
| | 0.05 | 0.10 | 0.25 | 0.5 | 1.0 | 40.0 |
| HU-235 (3a) | 44.4 (N.S.) | 38.7* | 31.9* | 60.1* | - | - |
| HU-245 (3c) | | 37.3* | 66.9* | 72.0* | - | - |
| 7b | | 39.0* | 59.0* | 68.2* | - | - |
| HU-211 (1a) | | - | - | 37.5 | 21.9 | - |
| | | | | (N.S.) | (N.S.) | |
| Δ⁶-THC-7-oic acid | | - | - | - | - | 50.2 |

| | | | | | | |
|---|---|---|---|---|---|---|
| ³Values shown are percent inhibition of paw edema when compared to vehicle treated controls. | | | | | | |
| *95% significance by ANOVA. N.S. - nonsignificant. | | | | | | |
| ⁴Control mice were given peanut oil (50 µl) orally). | | | | | | |

### 2. Decreased Adhesion of Leukocytes.

Leukocytes are thought to be major contributors to the inflammatory response and their ability in this regard is reflected by their adhesiveness to a variety of substrates [Audette, C.A., et al., Life Sci., 47, 753 (1990)]. The data in Table III show that peritoneal leukocytes from mice orally administered with the present cannabinoids exhibit decreased adhesion.

The details of the leukocytes adhesion assay have been previously reported [Audette et al., ibid.]. In brief, peritoneal cells from femals CD-1 mice (20-25 g) were collected at 90 min following oral administration of the drug or vehicle (50 µl peanut oil). Cells from each treatment group (N=3) were pooled and equal numbers aliquoted into six culture dish wells (1.9 cm² area). After incubation for 18-20 hours, non-adhering cells were removed and the remaining cell monolayer quantitated by DNA measurement.

**Table III**

| Effects of Leukocyte Adhesion⁵ | | | |
|---|---|---|---|
| Dose (mg/kg)⁶ | HU-211(1a) | HU-245(3c) | 235(3a) |
| Control | 0.88±0.08(100) | 1.26±0.05(100) | 1.26±0.05(100) |
| 0.01 | - | - | 1.34±0.14(106) |
| 0.05 | 1.09±0.08(124)* | 1.32±0.04(105) | 1.29±0.05(102) |
| 0.10 | 0.44±0.03(50)* | 0.66±0.05(52)* | 1.38±0.17(110) |
| 0.20 | - | - | - |
| 0.50 | 0.64±0.06(73)* | 0.85±0.08(67)* | 1.46±0.05(116) |
| 1.00 | 0.59±0.06(67)* | 0.30±0.04(24)* | 0.70±0.12(56)* |
| THC-7-oic Acid | | | |
| Control | 0.81±0.03- | | |
| 20 | 0.67±0.02(90.6)* | | |
| 40 | 0.55±0.22(67.9)* | | |
| 3. Antinociception. | | | |

| | | | |
|---|---|---|---|
| ⁵Values are the number of adhering cells x 10⁻⁶ ± S.D. Numbers in parentheses are percent of control. For details see Experimental Section. | | | |
| *95% significance by ANOVA; otherwise not statistically significant. | | | |
| ⁶Control mice were given 50 µl peanut oil orally. Peritoneal cells were collected 90 min after oral administration of cannabinoids. | | | |

### 3. Antinociception.

As with many NSAID type drugs, the present cannabinoids also showed activity in the mouse hot plate test (55°C) for antinociception.

In the hot plate test for analgesia an aluminum surface was maintained at 55 ± 1°C by circulating water through passages in the metal. A clear plastic cylinder 18 cm in diameter and 26 cm high was placed on the surface to prevent escape. The end point was taken when the mouse either performed a hind paw lick or jumped off the surface; in no case were the animals kept for more than 30 secs on the plate. Mice were never used more than one time; control values were always measured at 11 a.m. and test values at 2 p.m. The drugs were administered orally 90 min before the hot plate test. The percent change in response time (latency) was calculated by comparing the mean of the control values with the mean of the test values and statistical signficance determined by a paired t-test analysis using software (Statview, 512) from Brainpower, Inc., Calabasas, Ca.

**Table IV**

| ANTINOCLOPCEPTIVE EFFECTS⁷ | | | | |
|---|---|---|---|---|
| Dose (mg/kg) | HU211 (1a) | 3e | HU-245(3c) | HU-235(3a) |
| 0.025 | - | - | 10.7(5) | 10.3(5) |
| 0.050 | - | - | 66.2(5)* | 61.7(5)* |
| 0.10 | - | - | 62.2(5)* | 49.5(20)* |
| 0.25 | 30.0(5)* | 10.4(5) | 68.1(10)* | 61.5(17)** |
| 0.50 | 72.5(5)*** | 49.0(10)* | 49.9(5)* | 51.7(8)* |
| 1.0 | -10.2(5) | 61.4(15)* | 12.5(5) | 14.7(5) |
| 2.0 | - | 37.5(10) | | - |
| 4.0 | - | 3.1(10) | - | - |

| | | | | |
|---|---|---|---|---|
| ⁷Values are the percent change in latency. See above for details. Figures in brackets are the number of mice. | | | | |
| *P<0.05; | | | | |
| **P<0.005 by a paired t test; otherwise not statistically significant. | | | | |

### 4. Cataleptic Effects.

The lack of CNS activity for the compounds described herein may be seen from the data shown in Table V. This was measured by the so-called "ring test" [Pertwee, R.G., et al., J. Pharmacol., 46, 753 (1972)] in which the cataleptic effects of cannabinoids can be quantitated. The compounds produced little or no response when compared with the parent drug.

The cataleptic response was measured using the ring test. Mice were placed on a horizontal wire ring 5.5 cm in diameter, which was attached to a 16 cm vertical rod. The hind paws and fore paws were placed at opposite sides of the ring. It is important that the ambient temperature is maintained at 30°C and that the environment be free of auditory stimuli and bright lights. The criteria for immobility are detailed by Pertwee. The response is calculated as the fraction of time the mouse is immobile over a 5-min test period. Measurements were always done between 2 and 4 p.m. and the animals were used only once.

**Table V**

| CATALEPTIC EFFECTS IN THE MOUSE⁸ | | |
|---|---|---|
| Treatment | Dose (mg/kg) | Response ± SD |
| Vehicle⁹ | - | 7.7 ± 4.4 |
| HU-245 (3c) | 0.5 | 6.8 ± 2.4 |
| HU-245 (3c) | 1.0 | 12.0 ± 6.0 |
| HU-235 (3a) | 0.25 | 12.3 ± 10.3 |
| HU-235 (3a) | 0.5 | 13.8 ± 7.9 |
| HU-235 (3a) | 1.0 | 10.4 ± 10.6 |
| HU-235 (3a) | 4.0 | 8.7 ± 5.6 |
| Δ¹-THC | 40 | 48.9 ± 16* |

| | | |
|---|---|---|
| ⁸The values are expressed as the means of the fraction of time the mice remained immobile ± SD. See above for other details. | | |
| *95% signficance by ANOVA; otherwise not statistically significant. | | |
| ⁹Peanut oil (50 µl) given orally. | | |

### 5. Neuroprotection.

The present compounds are active in several tests generally used to indicate neuroprotection. For example, HU-245 which was investigated most thoroughly, is a blocker of N-methyl-D-aspartate (NMDA) and picrotoxin induced lethality in mice, as seen previously for HU-211 (compound la) (Israel Patent Application No. 92238). It also protects against strychnine induced lethality in rats. Compounds HU-211 (la), HU-235 (3a) and HU-245 (3c) have also been shown to protect against forebrain ischemia (unilateral occlusion) in mongolian gerbils.

Balb/c male mice were administered with the drug 90 min before treatment with NMDA (125 mg/kg) or 120 min before picrotoxin (8 mg/kg) in emulsion, s.c. The survival rate shown in Table VI indicates number of survivors out of total number of animals used.

**Table VI**

| Toxin | Drug | Survival rate |
|---|---|---|
| Picrotoxin | - | 0/17 |
| Picrotoxin | HU-245 (10 mg/kg) | 5/8 |
| Picrotoxin | HU-211 (10 mg/kg) | 6/10 |
| Picrotoxin | HU-235 (10 mg/kg) | 8/12 |
| NMDA | - | 0/20 |
| NMDA | HU-245 (2.5 mg/kg) | 6/12 |
| NMDA | HU-245 (10 mg/kg) | 8/10 |
| NMDA | HU-235 (10 mg/kg) | 7/10 |
| NMDA | HU-211 (10 mg/kg) | 11/16 |

### 6. Anti-Glaucoma Activity.

Reduction of intraocular pressure in rabbits was determined by the addition of a 50 µl drop of a 1% emulsion of the cannabinoid into the rabbit eye. This test is a measure of anti-glaucomatic activity. Significant reduction was established for compounds HU-235 and Hu-245.

### 7. NMDA-blocking Response in Oocytes.

The oocyte is the most widely used system for studying the molecular, biophysical and pharmacological properties of expressed voltage- or transmitter-operated ion channels. This giant cell offers several advantages for this type of studies. It can be readily used for two-electrode voltage clamp studies, translates injected mRNA from different sources and functionally expresses the translated proteins, and it is also readily amenable for intracellular microinjections. Importantly, the recordings of transmitter- or voltage-incuded currents in a single oocyte are extremely stable and can be done, without rundown, for 2-3 hours; this allows the performance of a full dose-response relation in single oocytes.

Neuronal receptors for excitatory amino acid (EAA) are becoming increasingly important for the understanding of normal and pathological brain function. Quantitative pharmacological studies of EAA receptor/ion channel complex are difficult to study in neurons. Therefore, Xenopus oocyte injected with brain mRNA is now the preparation of choice.

The response to N-methyl-D-aspartate (NMDA) in oocytes injected with rat brain mRNA was characterized and the effects of HU-245 were studied.

Female Xenopus were anesthetized by immersion in water containing 0.15% tricaine methanesulfonate and a small incision was made in the lower adbomen. Ovary fragments were removed into ND-96. The frog was allowed to heal for at least three months before using again.

The oocytes were defolliculated by the collagenase treatment in order to remove the follicle cell layer. Defolliculated oocytes were injected with total rat brain RNA and incubated for 2-4 days at 22°C in sterile NDE-96 solution. ND-96 solution contains (in mM): NaCl 96, KCl 2, CaCl₂ 1.8, Hepes 5 (pH=7.4-7.6). NDE-96 contains in addition, 2.5 mM sodium pyruvate, 100 unit/ml penicillin, 100 µg/ml streptomycin.

A single oocyte was placed in a 1 ml bath constantly superfused with ND-96 solution at room temperature. The cell was impeled with two microelectrodes and held at membrane potential of -60mV using voltage clamp circuit.

RNA was extracted from brain of 14-day old rat brain. Each oocyte was injected with 50 nl total RNA (4-8 mg/ml in water, stored at -80°C in 3-10 nl aliquots).

All chemicals were from Sigma.

Oocytes injected with rat brain RNA became responsive to all EAA (NMDA, kainate, glutamate) and to other transmitters (5HT, GABA, etc.). The conductance activated by NMDA was examined more closely. In most oocytes NMDA alone did not evoke any detectable currents, and the addition of glycine was necessary in order to evoke a response. Therefore, to observe an NMDA response, the combination: 10-⁴ M NMDA AND 5x10-⁶ M glycine was used in all the experiments.

Several NMDA receptor blockers were tested. These compounds have very low solubility and have to be dissolved in 100% dimethyl sulfoxide (DMSO) and diluted for use in ND 96 that contained 1% DMSO. Therefore, the effect of 1% DMSO on the response to 10-⁴ M NMDA and 5x10-⁶ M glycine was examined. DMSO had no effect on NMDA response in three different cells.

The new derivative HU-245 was tested on the NMDA response at a concentration of 10⁻⁶ M. A complete blocking effect could not be achieved since the compound is only soluble at a concentration of up to 10-⁷ M. Compound HU-245 had a strong inhibitory effect on the response to NMDA - 55% of control at 10-^{6M}. Another advantage of HU-245 seems to be its solubility, which is higher than that of prior art compounds. The effect of HU-245 was reversible upon return to ND 96 solution.

## Claims

1. A compound of the formula: 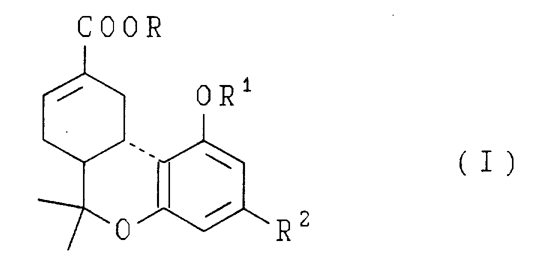 wherein R is a hydrogen or a C₁-C₅ alkyl group, R¹ is a hydrogen atom or a C₁-C₅ acyl or alkyl group and R² is selected from the group consisting of: (a) a straight-chained or branched C₅-C₁₂ alkyl radical; (b) a group -O-R⁴, where R⁴ is a straight-chained or branched C₂-₉ alkyl radical which may be substituted at the terminal carbon atom by a phenyl group; and (c) a group -(CH₂)ₙ-O-alkyl, where n is an integer of from 1 to 7 and the alkyl group contains from 1 to 5 carbon atoms, with the exception of 6a, 7, 10, 10a-tetrahydro-6,6-dimethyl-9-carboxyl-3-pentyl-6H-dibenzo[b,d]pyran-l-ol; the said compound of formula (I) having the (3S,4S) configuration, essentially free of the (3R,4R) enantiomer.

2. A compound according to claim 1 wherein R is hydrogen and R¹ is hydrogen or methyl.

3. A compound according to claim 1 or claim 2 wherein R² is 1,1-dimethylheptyl.

4. A compound of general formula (I) being the 1,1-dimethylheptyl homolog of (+)-(3S,4S)-delta-6-tetrahydrocannabinol-7-oic acid.

5. A compound of general formula (I) being the 1,1-dimethylheptyl homolog of (3S,4S)-(+) -delta-6-tetrahydrocannabinol acetate-7-oic acid.

6. A process for the preparation of a compound of formula (I) as defined in claim 1 comprising:
(a) reacting a compound of the formula: 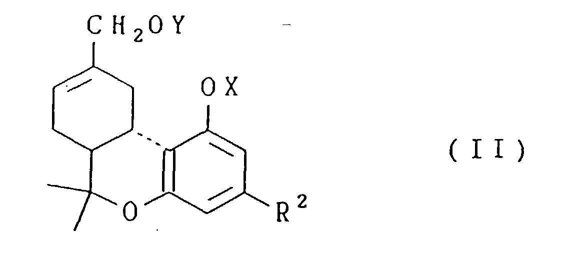 wherein Y is a straight chain or branched C₁ to C₅ alkyl group, X is a suitable protective group and R² is as defined in claim 1 with a suitable reducing agent to give the corresponding allylic alcohol of formula III: 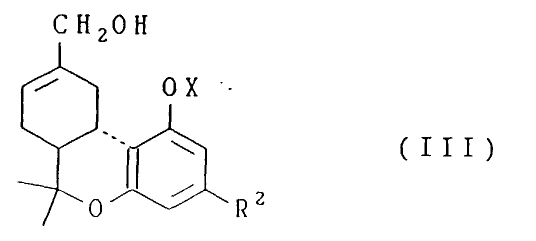
(b) oxidizing the alcohol of formula III with a suitable oxidizing agent to give the corresponding aldehyde of formula IV: 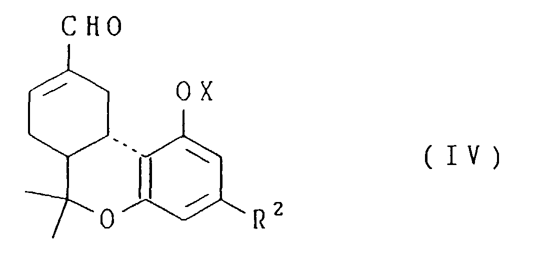
(c) oxidizing the aldehyde of formula IV with a suitable oxidizing agent to give the corresponding allylic acid of formula V: 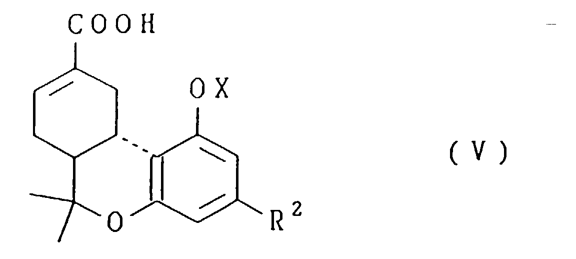 and
(d) removing the protective group X to give the acid according to formula (I).

7. A process according to claim 6 wherein Y is a (tri-methyl)methyl group.

8. A process according to claim 6 wherein the group -COOY is replaced by the group -CONR⁴R⁵ group wherein R⁴ and R⁵ are C₁ to C₅ alkyl groups.

9. A process according to claim 6 wherein X is a protective group selected from dimethyl-tert.-butylsilyl, dimethyl phenylsilyl, C₁-C₅ alkyl or benzyl.

10. A process according to claim 9 wherein X is dimethyl-tert.-butylsilyl.

11. A process according to claim 6 wherein said reducing agent is lithium aluminum hydride.

12. A process for the preparation of a compound of general formula (I), as defined in claim 1, comprising:
(a) subjecting a compound of the formula: 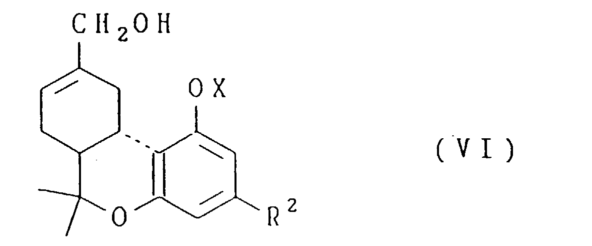 wherein R² is as defined for R² in claim 1 to selective esterification, to give the corresponding phenolic ester of formula VII: 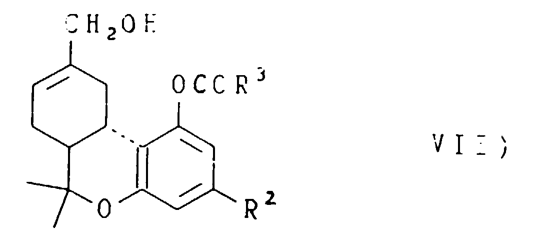 wherein R³ is a C¹-C⁵ alkyl, or benzyl which may be substituted at the para position by chlorine or bromine;
(b) oxidizing the ester of formula VII with a suitable oxidizing agent to give the corresponding aldehyde of formula VIII: 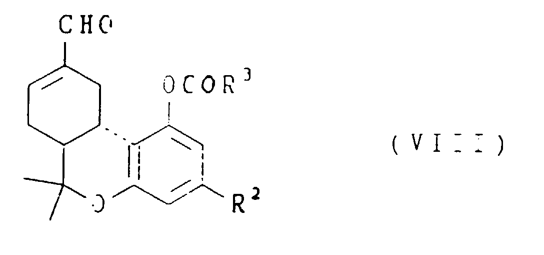
(c) oxidizing the aldehyde of formula VIII with a suitable oxidizing agent to give the corresponding acid of formula I: 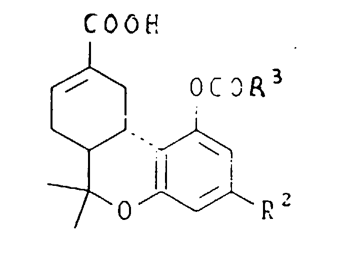 and
optionally removing the ester group COR³ to give the corresponding compound:

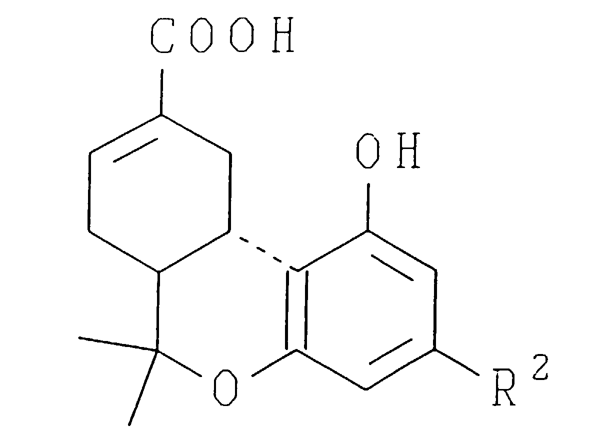

13. A process according to any one of claims 6 to 12 wherein the oxidizing agent in step (b) is chromic oxide, tert.-butyl chromate, chromic acid/pyridine complex or manganese dioxide.

14. A process according to any one of claims 6 to 13 wherein the oxidizing agent in step (c) is sodium chlorite or manganese dioxide.

15. A pharmaceutical composition comprising as active ingredient a compound of general formula (I) as defined in claim 1.

16. A pharmaceutical composition according to claim 16 wherein the active ingredient is the 1,1-dimethyl-heptyl homolog of (3S,4S)-(+)-delta-6-tetrahydrocannabinol-7-oic acid.

17. A pharmaceutical composition according to claim 16 wherein the active ingredient is the 1,1-dimethyl-heptyl homolog of (3S,4S)-(+)-delta-6-tetrahydrocannabinol-acetate-7-oic acid.

18. A pharmaceutical composition according to any one of claims 15 to 17 which is:
(a) analgesic;
(b) anti-inflammatory;
(c) anti-emetic;
(d) anti-glaucoma;
(e) has leukocyte antiadhesion activity; or
(f) has anti-platelet activity factor (PAF) activity.

19. A pharmaceutical composition according to any one of claims 15 to 17 for:
(a) the treatment of accute injuries to the central nervous system due to excitatory amino acid neuro-toxicity, optionally for the treatment of prolonged epileptic seizures, compromised or reduced blood supply, deprivation of glucose supply, mechanical trauma, global hypoxic ischemic insults, cardiac arrest or stroke;
(b) the treatment of chronic degenerative disease associated with gradual selective neuronal loss, optionally for the treatment of Huntington's chorea, Parkinsonism, Alzheimer's disease and multiple sclerosis; or
(c) the treatment of poisoning affecting the central nervous system, optionally for treating strychnine, picrotoxin or oraganophosphorous poisoning.

20. Pharmaceutical compositions according to any one of claims 15 to 17 in dosage unit form.

21. A compound of formula (I) as defined in claim 1 for use as a medicament.

22. A compound of formula (I) as defined in claim 1 for use in treating inflammation, pain or glaucoma, vomiting or disease states due to platelet activating factor irregularities or faulty leukocyte adhesion activity; acute injuries to the central nervous system associated with excitatory amino acid neurotoxicity, optionally of prolonged epileptic seizures, compromised or reduced blood supply, deprivation of glucose supply, mechanical trauma, global hypoxic ischemic insults, cardiac arrest or stroke; chronic degenerative disease associated with gradual selective neuronal loss, optionally Huntington's chorea, Parkinsonism, Alzheimer's disease or multiple sclerosis. poisoning affecting the central nervous system, optionally for treating strychnine, picrotoxin or oraganophosphorous poisoning.

## Patentansprüche

1. Verbindung der Formel: 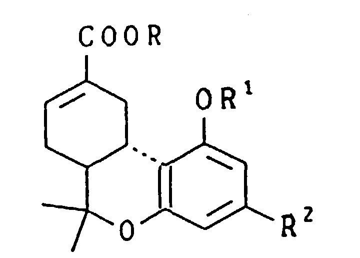 worin R Wasserstoff oder eine C₁-C₅-Alkylgruppe ist, R¹ ein Wasserstoffatom oder eine C₁-C₅-Acyl- oder -Alkylgruppe ist und R² ausgewählt ist aus der Gruppe, bestehend aus: (a) einem geradkettigen oder verzweigten C₅-C₁₂-Alkylrest; (b) einer Gruppe -O-R⁴, worin R⁴ ein geradkettiger oder verzweigter C₂₋₉-Alkylrest ist, der am terminalen Kohlenstoffatom mit einer Phenylgruppe substituiert sein kann; und (c) einer Gruppe -(CH₂)ₙ-O-Alkyl, worin n eine ganze Zahl von 1 bis 7 ist und die Alkylgruppe von 1 bis 5 Kohlenstoffatome enthält, mit der Ausnahme von 6a,7,10,10a-Tetrahydro-6,6-dimethyl-9-carboxyl-3-pentyl-6H-dibenzo[b,d]pyran-1-ol, wobei die Verbindung von Formel (I) die (3S,4S)-Konfiguration aufweist und im wesentlichen frei vom (3R,4R)-Enantiomer ist.

2. Verbindung nach Anspruch 1, worin R Wasserstoff ist und R¹ Wasserstoff oder Methyl ist.

3. Verbindung nach Anspruch 1 oder Anspruch 2, worin R² 1,1-Dimethylheptyl ist.

4. Verbindung der allgemeinen Formel (I), welche das 1,1-Dimethylheptyl-Homologe der (+)-(3S,4S)-delta-6-Tetrahydrocannabinol-7-säure ist.

5. Verbindung der allgemeinen Formel (I), welche das 1,1-Dimethylheptyl-Homologe der (3S,4S)-(+)-delta-6-Tetrahydrocannabinolacetat-7-säure ist.

6. Verfahren zur Herstellung einer Verbindung der Formel (I), wie in Anspruch 1 definiert, umfassend:
(a) Umsetzen einer Verbindung der Formel: 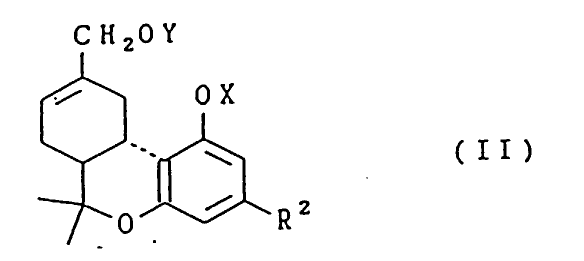 worin Y eine geradkettige oder verzweigte C₁-C₅-Alkylgruppe ist, X eine geeignete Schutzgruppe ist und R² wie in Anspruch 1 definiert ist, mit einem geeigneten Reduktionsmittel, um den entsprechenden Allylalkohol der Formel III zu ergeben: 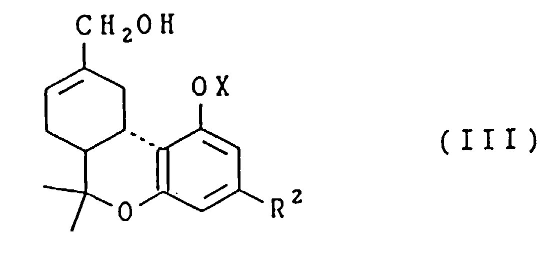
(b) Oxidieren des Alkohols der Formel III mit einem geeigneten Oxidationsmittel, um den entsprechenden Aldehyd der Formel IV zu ergeben: 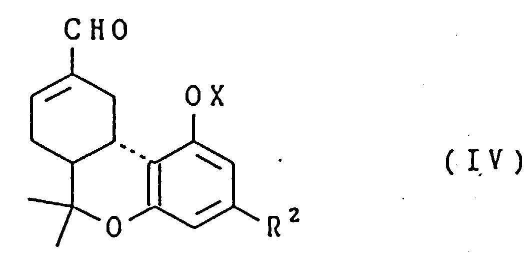
(c) Oxidieren des Aldehyds der Formel IV mit einem geeigneten Oxidationsmittel, um die entsprechende Allylsäure der Formel V zu ergeben: 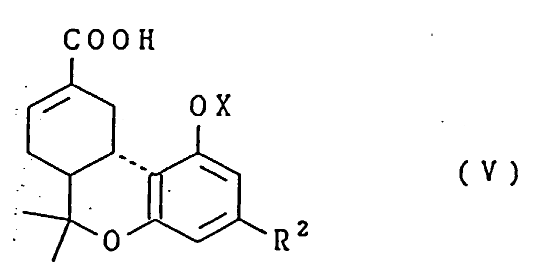 und (d) Entfernen der Schutzgruppe X, um die Säure gemäß Formel (I) zu ergeben.

7. Verfahren nach Anspruch 6, worin Y eine (Trimethyl)methyl-Gruppe ist.

8. Verfahren nach Anspruch 6, worin die Gruppe -COOY durch die Gruppe -CONR⁴R⁵ ersetzt wird, worin R⁴ und R⁵ C₁-C₅-Alkylgruppen sind.

9. Verfahren nach Anspruch 6, worin X eine Schutzgruppe ist, ausgewählt aus Dimethyl-tert-butylsilyl, Dimethylphenylsilyl, C₁-C₅-Alkyl oder Benzyl.

10. Verfahren nach Anspruch 9, worin X Dimethyl-tert-butylsilyl ist.

11. Verfahren nach Anspruch 6, worin das Reduktionsmittel Lithiumaluminiumhydrid ist.

12. Verfahren zur Herstellung einer Verbindung der allgemeinen Formel (I), wie in Anspruch 1 definiert, umfassend:
(a) Unterziehen einer Verbindung der Formel: 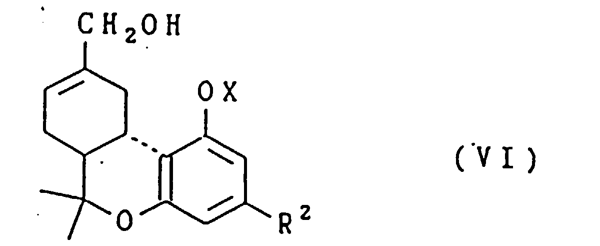 worin R² wie für R² in Anspruch 1 definiert ist, einer selektiven Veresterung, um den entsprechenden Phenolester der Formel Vll zu ergeben: 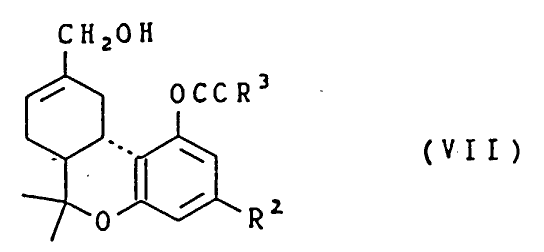 worin R³ ein C₁-C₅-Alkyl oder ein Benzyl ist, das an der Paraposition mit Chlor oder Brom substituiert sein kann;
(b) Oxidieren des Esters der Formel Vll mit einem geeigneten Oxidationsmittel, um den entsprechenden Aldehyd der Formel Vlll zu ergeben: 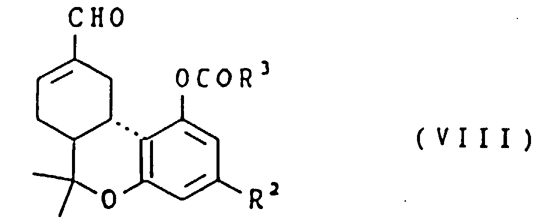
(c) Oxidieren des Aldehyds der Formel Vlll mit einem geeigneten Oxidationsmittel, um die entsprechende Säure der Formel 1 zu ergeben: 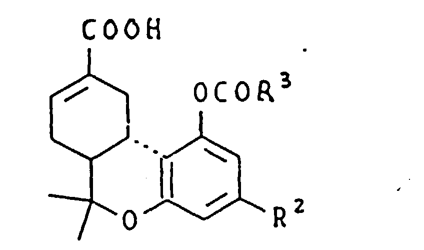 und gegebenenfalls Entfernen der Estergruppe COR³, um die entsprechende Verbindung: 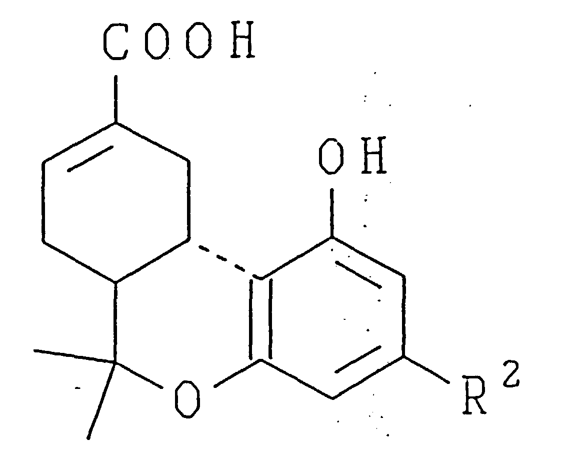 zu ergeben.

13. Verfahren nach einem der Ansprüche 6 bis 12, worin das Oxidationsmittel in Schritt (b) Chromoxid, tert-Butylchromat, ein Chromsäure/Pyridin-Komplex oder Mangandioxid ist.

14. Verfahren nach einem der Ansprüche 6 bis 13, worin das Oxidationsmittel in Schritt (c) Natriumchlorit oder Mangandioxid ist.

15. Pharmazeutische Zusammensetzung, umfassend als Wirkstoff eine Verbindung der allgemeinen Formel (I), wie in Anspruch 1 definiert.

16. Pharmazeutische Zusammensetzung nach Anspruch 15, worin der Wirkstoff das 1,1-Dimethyl-heptyl-Homologe der (3S,4S)-(+)-delta-6-Tetrahydrocannabinol-7-säure ist.

17. Pharmazeutische Zusammensetzung nach Anspruch 15, worin der Wirkstoff das 1,1-Dimethyl-heptyl-Homologe der (3S,4S)-(+)-delta-6-Tetrahydrocannabinol-acetat-7-säure ist.

18. Pharmazeutische Zusammensetzung nach einem der Ansprüche 15 bis 17, welche:
(a) schmerzlindernd ist;
(b) entzündungshemmend ist;
(c) anti-emetisch ist;
(d) anti-glaukomisch ist;
(e) eine Leukozyten-anti-Adhäsionsaktivität aufweist; oder
(f) eine anti-Plättchen-aktivierender Faktor (PAF)-Aktivität aufweist.

19. Pharmazeutische Zusammensetzung nach einem der Ansprüche 15 bis 17 für:
(a) die Behandlung von akuten Verletzungen des zentralen Nervensystems aufgrund einer exzitatorischen Aminosäuren-Neurotoxizität, gegebenenfalls für die Behandlung von anhaltenden epileptischen Anfällen, beeinträchtigter oder verringerter Blutzufuhr, Glucosezufuhrmangel, mechanischen Traumata, globalen hypoxischen ischämischen Verletzungen, Herzstillstand oder -schlag;
(b) die Behandlung einer chronischen degenerativen Erkrankung verbunden mit einem schrittweisen selektiven Neuronenverlust; gebenenfalls für die Behandlung von Chorea Huntington, Parkinson, Alzheimer-Erkrankung und Multiple Sklerose; oder
(c) die Behandlung einer Vergiftung, die das zentrale Nervensystem beeinflußt, gegebenfalls für die Behandlung einer Vergiftung durch Strychnin, Picrotoxin oder Organophosphor.

20. Pharmazeutische Zusammensetzung nach einem der Ansprüche 15 bis 17 in Form einer Dosiseinheit.

21. Verbindung der Formel (I), wie in Anspruch 1 definiert, zur Verwendung als Medikament.

22. Verbindung der Formel (I), wie in Anspruch 1 definiert, zur Behandlung einer Entzündung, von Schmerz oder eines Glaukoms, von Erbrechen oder Erkrankungszuständen aufgrund von Unregelmäßigkeiten beim Plättchen-aktivierenden Faktor oder einer fehlerhaften Leukozytenadhäsionsaktivität, akuten Verletzungen des zentralen Nervensystems, verbunden mit einer exzitatorischen Aminosäuren-Neurotoxizität, gegebenenfalls von anhaltenden epileptischen Anfällen, beeinträchtigter oder verringerter Blutzufuhr, Glucosezufuhrmangel, mechanischen Traumata, globalen hypoxischen ischämischen Verletzungen, Herzstillstand oder -schlag; chronisch degenerativen Erkrankungen verbunden mit einem schrittweisen selektiven Neuronenverlust, gegebenenfalls Chorea Huntington, Parkinson, Alzheimer-Erkrankung oder Multipler Sklerose, einer Vergiftung, die das zentrale Nervensystem beeinflußt, gegebenfalls zur Behandlung einer Vergiftung durch Strychnin, Picrotoxin oder Organophosphor.

## Revendications

1. Composé de formule : 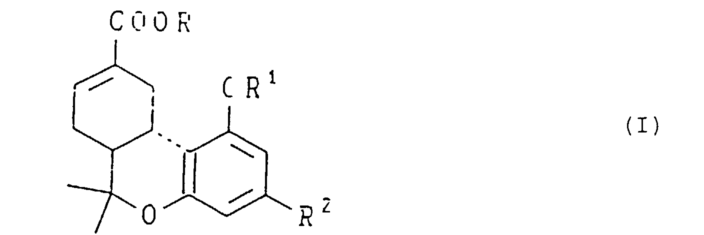
dans laquelle R est un atome d'hydrogène ou un groupe alkyle en C₁ à C₅, R¹ est un atome d'hydrogène ou un groupe acyle ou alkyle en C₁ à C₅ et R² est choisi dans l'ensemble constitué par : (a) un groupe alkyle en C₅ à C₁₂ à chaîne linéaire ou ramifiée ; (b) un groupe -O-R₄ où R₄ est un groupe alkyle en C₂ à C₉ à chaîne linéaire ou ramifiée qui peut être substitué sur l'atome de carbone terminal par un groupe phényle ; et (c) un groupe-(CH₂)ₙ-O-alkyle où n est un entier de 1 à 7 et le groupe alkyle contient de 1 à 5 atomes de carbone,
à l'exception du 6a,7,10,10a-tétrahydro-6,6-diméthyl-9-carboxy-3-pentyl-6H-dibenzo[b,d]pyrane-1-ol ;
ledit composé de formule (I) ayant la configuration (3S,4S), pratiquement exempte de l'énantiomère (3R,4R).

2. Composé selon la revendication 1, dans lequel R est l'hydrogène et R¹ est l'hydrogène ou le groupe méthyle.

3. Composé selon la revendication 1 ou la revendication 2, dans lequel R² est le groupe 1,1-diméthylheptyle.

4. Composé de formule générale (I), qui est l'homologue 1,1-diméthylheptylique d'acide (+)-(3S,4S)-delta-6-tétrahydrocannabinol-7-oïque.

5. Composé de formule générale (I), qui est l'homologue 1,1-diméthylheptylique d'acide (+)-(3S,4S)-delta-6-tétrahydrocannabinol-7-oïque sous forme acétate.

6. Procédé pour la préparation d'un composé de formule (I) tel que défini dans la revendication 1, comprenant :
(a) la réaction d'un composé de formule : 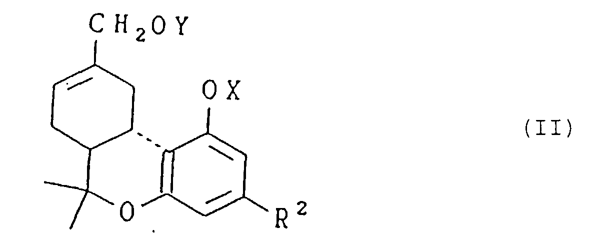 dans laquelle Y est un groupe alkyle en C₁ à C₅ à chaîne linéaire ou ramifiée, X est un groupe protecteur convenable et R² est tel que défini dans la revendication 1,
avec un agent réducteur convenable, pour donner l'alcool allylique correspondant de formule III : 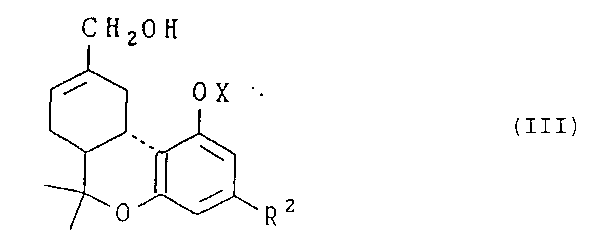
(b) l'oxydation de l'alcool de formule III avec un agent oxydant convenable pour donner l'aldéhyde correspondant de formule IV : 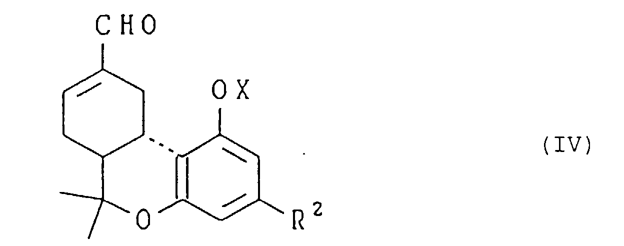
(c) l'oxydation de l'aldéhyde de formule IV avec un agent oxydant convenable pour donner l'acide allylique correspondant de formule V : 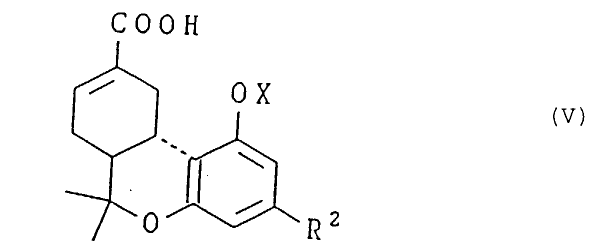 et
(d) l'élimination du groupe protecteur X pour donner l'acide selon la formule (I).

7. Procédé selon la revendication 6, dans lequel Y est un groupe (triméthyl)méthyle.

8. Procédé selon la revendication 6, dans lequel le groupe -COOY est remplacé par le groupe -CONR⁴R⁵ où R⁴ et R⁵ sont des groupes alkyle en C₁ à C₅.

9. Procédé selon la revendication 6, dans lequel X est un groupe protecteur choisi parmi les groupes diméthyl-tert-butylsilyle, diméthylphénylsilyle, alkyle en C₁ à C₅ et benzyle.

10. Procédé selon la revendication 9, dans lequel X est le groupe diméthyl-tert-butylsilyle.

11. Procédé selon la revendication 6, dans lequel ledit agent réducteur est l'hydrure de lithium et d'aluminium.

12. Procédé pour la préparation d'un composé de formule générale (I), tel que défini dans la revendication 1, comprenant :
(a) la soumission d'un composé de formule : 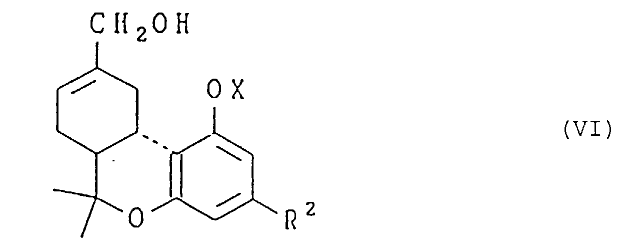 dans laquelle R² est tel que défini pour R² dans la revendication 1,
à une estérification sélective, pour donner l'ester phénolate correspondant de formule VII : 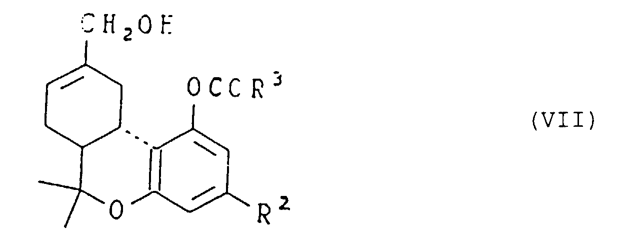 dans laquelle R³ est un groupe alkyle en C₁ à C₅ ou benzyle qui peut être substitué en position para par du chlore ou du brome ;
(b) l'oxydation de l'ester de formule VII avec un agent oxydant convenable pour donner l'aldéhyde correspondant de formule VIII : 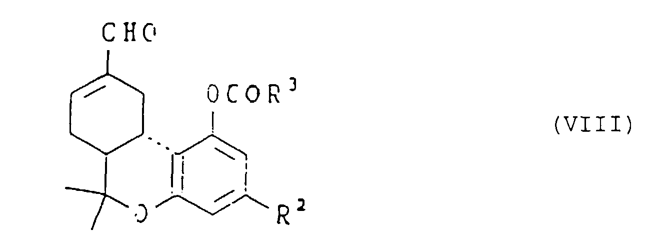
(c) l'oxydation de l'aldéhyde de formule VIII avec un agent oxydant convenable pour donner l'acide correspondant de formule I : 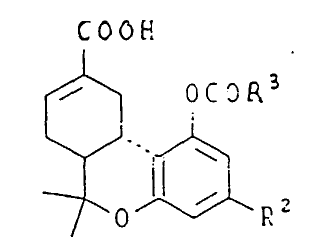 et
éventuellement élimination du groupe ester COR³ pour donner le composé correspondant :

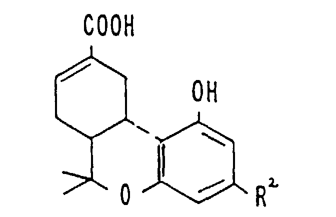

13. Procédé selon l'une quelconque des revendications 6 à 12, dans lequel l'agent oxydant dans l'étape (b) est l'oxyde chromique, le chromate de tert-butyle, un complexe acide chromique/pyridine ou le dioxyde de manganèse.

14. Procédé selon l'une quelconque des revendications 6 à 13, dans lequel l'agent oxydant dans l'étape (c) est le chlorite de sodium ou le dioxyde de manganèse.

15. Composition pharmaceutique comprenant, à titre d'ingrédient actif, un composé de formule générale (I) tel que défini dans la revendication 1.

16. Composition pharmaceutique selon la revendication 16, dans laquelle l'ingrédient actif est l'homologue 1,1-diméthylheptylique d'acide (+)-(3S,4S)-delta-6-tétrahydrocannabinol-7-oïque.

17. Composition pharmaceutique selon la revendication 16, dans laquelle l'ingrédient actif est l'homologue 1,1-diméthylheptylique d'acide (+)-(3S,4S)-delta-6-tétrahydrocannabinol-7-oïque sous forme acétate.

18. Composition pharmaceutique selon l'une quelconque des revendications 15 à 17, qui :
(a) est analgésique ;
(b) est anti-inflammatoire ;
(c) est antiémétique ;
(d) est anti-glaucome ;
(e) a une activité d'anti-adhérence des leucocytes ; ou
(e) a une activité d'anti-facteur activant les plaquettes (PAF).

19. Composition pharmaceutique selon l'une quelconque des revendications 15 à 17, pour :
(a) le traitement de lésions aiguës du système nerveux central dues à une neurotoxicité par des acides aminés excitateurs, éventuellement pour le traitement de crises épileptiques prolongées, d'un apport de sang compromis ou réduit, d'une privation d'apport de glucose, d'un traumatisme mécanique, d'accidents ischémiques hypoxiques globaux, d'un arrêt cardiaque ou d'un ictus ;
(b) le traitement d'une maladie dégénérative chronique associée à une perte neuronale sélective graduelle, éventuellement pour le traitement de la chorée de Huntington, du parkinsonisme, de la maladie d'Alzheimer et de la sclérose en plaques ; ou
(c) le traitement d'un empoisonnement affectant le système nerveux central, éventuellement pour le traitement d'un empoisonnement à la strychnine, à la picrotoxine ou avec un composé organique du phosphore.

20. Compositions pharmaceutiques selon l'une quelconque des revendications 15 à 17, sous forme posologique unitaire.

21. Composé de formule (I) selon la revendication 1 pour une utilisation en tant que médicament.

22. Composé de formule (I) tel que défini dans la revendication 1, à utiliser dans le traitement d'une inflammation, d'une douleur ou d'un glaucome, d'états émétiques ou morbides dus à des irrégularités du facteur activant les plaquettes ou à une activité d'adhérence des leucocytes aberrante ; de lésions aiguës du système nerveux central associées à une neurotoxicité vis-à-vis d'acides aminés excitateurs, éventuellement de crises épileptiques prolongées, d'un apport de sang compromis ou réduit, d'une privation d'apport de glucose, d'un traumatisme mécanique, d'accidents ischémiques hypoxiques globaux, d'un arrêt cardiaque ou d'un ictus ; d'une maladie dégénérative chronique associée à une perte neuronale sélective graduelle, éventuellement de la chorée de Huntington, du parkinsonisme, de la maladie d'Alzheimer et de la sclérose en plaques ; ou d'un empoisonnement affectant le système nerveux central, éventuellement pour le traitement d'un empoisonnement à la strychnine, à la picrotoxine ou avec un composé organique du phosphore.
